# EUROPEAN PATENT APPLICATION

(11) **EP 4 652 850 A1**
(43) Date of publication of application: **26.11.2025**
(21) Application number: 24707253.1
(22) Date of filing: 17.01.2024
(51) Int. Cl.: A23C 9/14, A23C 9/15, A23J 1/20, A61K 8/14, A61K 9/127, A61K 35/20

(54) **PROCEDURE FOR OBTAINING EXOSOMES FROM BUTTERMILK, EXOSOMES OBTAINED AND THEIR APPLICATIONS**

(30) Priority: 17.01.2023 ES 202330029
(71) Applicant: Universidad Autónoma de Madrid, 28049 Madrid (ES); Fundación Imdea Alimentación, 28049 Madrid (ES)
(72) Inventor: MARÍN MARTÍN, Francisco Ramón, 28049 MADRID (ES); SEÑORANS RODRÍGUEZ, Francisco Javier, 28049 MADRID (ES); DÁVALOS HERRERA, Alberto, 28049 MADRID (ES); LÓPEZ DE LAS HAZAS MINGO, María del Carmen, 28049 MADRID (ES)
(74) Representative: Herrero & Asociados, S.L.
(86) International application number: PCT/ES2024/070031
(87) International publication number: WO 2024/153847

(57) **Abstract**

This invention contemplates a procedure to isolate exosomes from buttermilk that comprises: (a) selecting the buttermilk as a byproduct of the dairy industry, (b) subjecting it to a first centrifugation, under conditions between 10,000 g and 15,000 g, 1°C and 8°C, and 15 and 30 min, obtaining a fatty upper layer, an aqueous fraction, and a precipitate; (c) Subjecting the aqueous fraction to a second centrifugation, under conditions between 30,000 g and 35,000 g, 1 and 10°C and between 50 min and 1 hour and 20 min; obtaining a precipitate and a supernatant; (d) collecting the upper 75% of the supernatant; (e) concentrating or separating the exosomes from the supernatant fraction; and (f) filtering the fraction obtained to obtain a fraction enriched in exosomes. Likewise, the exosomes obtained by the procedure of the invention and their applications in medicine, cosmetics and nutraceuticals are contemplated.

## Description

### Field of invention

This invention is related, in general, to obtaining extracellular vesicles from byproducts of the dairy industry. In particular, the present invention contemplates a procedure for obtaining and isolating exosomes from buttermilk or whey obtained from the manufacture of butter, the exosomes obtained from this procedure and their applications in the field of nutrition, cosmetics and medicine.

### Background of the invention

Exosomes are extracellular vesicles of endocytic origin, with a variable size between 40-150 nm and a density between 1.15-1.19 g/mL present in all biological fluids *(*Théry, C., Amigorena, S., Raposo, G., Clayton, A. (2006). Isolation and characterization of exosomes from cell culture supernatants and biological fluids. Curr Protoc Cell Biol. Apr;Chapter 3:Unit 3.22. doi: 10.1002/0471143030.cb0322s30*).*

Morphologically they can be described as a lipid bilayer structure that constricts a lumen in which different compounds are found. The composition of the lipid bilayer (enriched in cholesterol, sphingomyelin, phosphatidylserine and saturated or monounsaturated fatty acids at the expense of phosphatidylcholine and ethanolamine) gives them particular characteristics such as low immunogenicity, low toxicity compared to liposomes, selectivity, as well as stability and protection of luminal content against enzymatic, chemical degradation and phagocytosis (Bell, B.M., Kirk, I.D., Hiltbrunner, S., Gabrielsson, S., Bultema, J.J. (2016). Designer exosomes as next-generation cancer immunotherapy. Nanomedicine. Jan;12(1):163-9. doi: 10.1016/j.nano.2015.09.011)*.* On the other hand, since the lipid bilayer comes mainly from the plasma membrane, they have proteins on the surface with diverse functions that include enzymatic, ligand and adhesion functions (Minotti, G., Menna, P., Salvatorelli, E., Cairo, G., Gianni, L. (2004). Anthracyclines: molecular advances and pharmacologic developments in antitumor activity and cardiotoxicity. Pharmacol Rev. Jun;56(2):185-229. doi: 10.1124/pr.56.2.6*).* Regarding the lumen, it contains different biological compounds that include lipids, proteins, and nucleic acids among which there can be found mRNAs, miRNAs, siRNAs, InRNAs and DNA fragments *(*Karlsson, O., Rodosthenous, R.S., Jara, C., Brennan, K.J., Wright, R.O., Baccarelli, A.A., Wright, R.J. (2016). Detection of long non-coding RNAs in human breastmilk extracellular vesicles: implications for early child development. Epigenetics. 11(10):721-9. https://doi.org/10.1080/15592294.2016.1216285*).*

Physiologically, exosomes seem to play an important role in intercellular and intertissue communication, being involved in processes of cellular reprogramming, metabolic regulation, cellular differentiation, neoplasia, migration, metastasis, immune response, interaction with the microbiome or viral immunity, among others *(*Kalluri, R., LeBleu, V.S. (2020). The biology, function, and biomedical applications of exosomes. Science. Feb 7;367(6478):eaau6977. doi: 10.1126/science.aau6977*).* The biological functions of exosomes, as regulators of intercellular and intertissue signaling pathways, make them potential vehicles for therapeutic agents in the control of numerous diseases, considering their use as nanocarriers for drugs, immunomodulators, nutrients or gene regulators (ie miRNAs, siRNAs, etc.), among others *(*Liao, W., Du, Y., Zhang, C., Pan, F., Yao, Y., Zhang, T., Peng, Q. (2019). Exosomes: The next generation of endogenous nanomaterials for advanced drug delivery and therapy. Acta Biomater. Mar 1;86:1-14. doi: 10.1016/j.actbio.2018.12.045*).* Thus, the use of exosomes as nanocarriers presents advantages, compared to other carrier agents (i.e., liposomes), such as greater efficiency in access to the cell, a lower immune response (Fitts, C.A., Ji, N., Li, Y., Tan, C. (2019). Exploiting exosomes in cancer liquid biopsies and drug delivery. Adv. Healthc. Mater. 8, e1801268 doi: 10.1002/adhm.201801268*),* and lower toxicity (M. Mendt, M., et al. (2018) Generation and testing of clinical-grade exosomes for pancreatic cancer. JCI Insight 3, e99263. doi: 10.1172/jci.insight.99263*).* Likewise, exosomes, both autologous and heterologous, can support a process of modification or enrichment of their surface ligands, which would allow greater specificity in the delivery of the therapeutic agent (Tran, P.H.L., Xiang, D., Tran, T.T.D., Yin, W., Zhang, Y., Kong, L., Chen, K., Sun, M., Li, Y., Hou, Y., Zhu, Y., Duan, W. (2020). Exosomes and Nanoengineering: A Match Made for Precision Therapeutics. Adv Mater. May;32(18):e1904040. doi: 10.1002/adma.201904040*).*

The properties mentioned in the previous paragraph have led to numerous clinical trials (Cully, M. (2021). Exosome-based candidates move into the clinic. Nat Rev Drug Discov. Jan;20(1):6-7. doi: 10.1038/d41573-020-00220-y*).* However, the use of exosomes has found some obstacles such as low production, low yield during isolation, considerable complexity and heterogeneity, lower specificity than desirable, and difficulty in the loading process of the cosmetic or nutritional therapeutic agent or active ingredients. Although approaches have been made based on the attempt to generate nanoparticles based on exosomes (i.e., totally artificial, modified from nanovesicles, hybrids between exosomes and nanovesicles, etc.), autologous and heterologous exosomes seem to be the most efficient, probably since they conserve their native biological properties intact (Lu, M., Huang, Y. (2020). Bioinspired exosome-like therapeutics and delivery nanoplatforms. Biomaterials. Mar 2;242:119925. doi: 10.1016/j.biomaterials.2020.119925*).*

The commercial use of exosomes requires transferring laboratory know-how to an industrial scale, which requires the development of scalable isolation protocols at an affordable cost. **In** this regard, the use of autologous exosomes, more suitable for precision medicine, faces the drawback of the extremely low concentrations that can be isolated. As an example, it is only possible to isolate quantities between 6-12 µg from 10⁶ dendritic cells (Alvarez-Erviti, L., Seow, Y., Yin, H. et al. (2011). Delivery of siRNA to the mouse brain by systemic injection of targeted exosomes. Nat Biotechnol 29, 341-345. https://doi.org/10.1038/nbt.1807)*,* with equally poor results found in the isolation of exosomes from human plasma (Lobb, R.J., Becker, M., Wen, S.W., Wong, C.S., Wiegmans, A.P., Leimgruber, A., Möller, A. (2015). Optimized exosome isolation protocol for cell culture supernatant and human plasma. J Extracell Vesicles. Jul 17;4:27031. doi: 10.3402/jev.v4.27031*).*

On the other hand, the release of exosomes is an evolutionarily conserved phenomenon in eukaryotic organisms, based on the studies carried out during the last 50 years (Yi, Y.W., Lee, J.H., Kim, S.Y., Pack, C.G., Ha, D.H., Park, S.R., Youn, J., Cho, B.S. (2020). Advances in Analysis of Biodistribution of Exosomes by Molecular Imaging. Int J Mol Sci. Jan 19;21(2):665. doi: 10.3390/ijms21020665*),* which allows the use of virtually any biological material, including industrial byproducts, as a potential source of heterologous exosomes. Thus, the presence of exosomes has been described both in foods and in the industrial byproducts of their production *(*Munir, J., Lee, M., Ryu, S. 2020. Exosomes in Food: Health Benefits and Clinical Relevance in Diseases. Adv Nutr. May 1;11(3):687-696. doi: 10.1093/advances/nmz123*).*

Among the above, the use of milk (and its byproducts) to obtain heterologous exosomes stands out, taking into account its nature as a biological fluid and the quality and abundance of these microvesicular structures. Thus, the presence of exosomes has been described in breast, bovine, caprine, porcine, equine and camelid milk *(*Adriano, B., Cotto, N.M., Chauhan, N., Jaggi, M., Chauhan, S.C., Yallapu, M.M. 2021. Milk exosomes: Nature's abundant nanoplatform for theranostic applications. Bioact Mater. Feb 2;6(8):2479-2490. doi: 10.1016/j.bioactmat.2021.01.009*).* These sources can produce tons of milk daily (*FAO.2022.https:*//*www.fao.org*/*dairy-production-products*/*production*/*en*/*#:~:text=In%20the %20last%20three%20decades,843%20mi llion%20tonnes%20in%202018. Accessed: 08*/*24*/*2022),* from which large quantities of exosomes can be obtained compared to the use of cell lines or other biological fluids (*Adriano B. et al. 2021*). On the other hand, from a technological point of view, exosomes derived from milk have a uniform particle size, around 100 nm in diameter (*Adriano B. et al. 2021*), and persist after industrial thermal treatments, freeze-thaw cycles, pH variations and even fermentation (Anusha, R., Priya, S. 2022. Dietary Exosome-Like Nanoparticles: An Updated Review on Their Pharmacological and Drug Delivery Applications. Mol Nutr Food Res. Jul;66(14):e2200142. doi: 10.1002/mnfr.202200142*),* which makes milk and its byproducts especially recommended as an industrial source of exosomes, and these, for their properties for therapeutic applications.

In this regard, numerous efforts have been made to develop methods and find sources for extracting exosomes from milk and its byproducts. Thus, different procedures for isolating exosomes, both from milk and colostrum, from one or different species (e.g. cow, sheep, goat, camel, horse, etc.) have been described (KR20220021136; JP2021153449; US2021228634; CN113061571; KR2021006675; CN113025561; WO2021202824; KR20200142785; WO2021092209; CN109468265). The procedures described consist of successive centrifugations, filtrations and precipitations (KR20220021136; JP2021153449; CN113061571; CN113025561; KR2021006675; CN11271092; WO2021202824; WO2021092209; CN109 468265; CN107375944).

The exosomes obtained from milk or whey have a characteristic composition and a particular content in miRNAs (US2021228634, WO2020218846, CN111012924, CN108315440, CN107375944) and can be exogenously loaded with different nutritional and/or therapeutic agents (US202122864, KR2 0220021136, CN113025561, CN112791092, US2021113622, KR20200142785, WO2020218846, WO2020018926, NZ728949).

However, it has been proven that milk and whey present some technical drawbacks that make obtaining exosomes difficult and expensive. Thus, raw milk has a high price due to its human consumption. Furthermore, in its raw state, it has a high fat content and a high heterogeneity in the size of the fat globules that require industrial operations for homogenization and skimming, with exosomes being isolated from the skimmed milk. For its part, the whey mostly used comes from cheese making, which involves an enzymatic treatment that can alter the surface proteins of the exosome.

Faced with the aforementioned drawbacks, the present invention is based on the development of an exosome extraction procedure in which a raw material not used until now for this purpose is used, such as buttermilk or whey obtained from the production of the butter. The characteristics of this raw material allow the application of a simpler extraction procedure than those used until now, by minimizing the degreasing operation, obtaining a higher performance than other industrial sources. Furthermore, the pH of this starting product is around 5, thus facilitating the elimination of some globular proteins, which facilitates the isolation and subsequent purification of exosomes, obtaining purer exosomes with fewer protein contaminants.

### Brief description of the figures

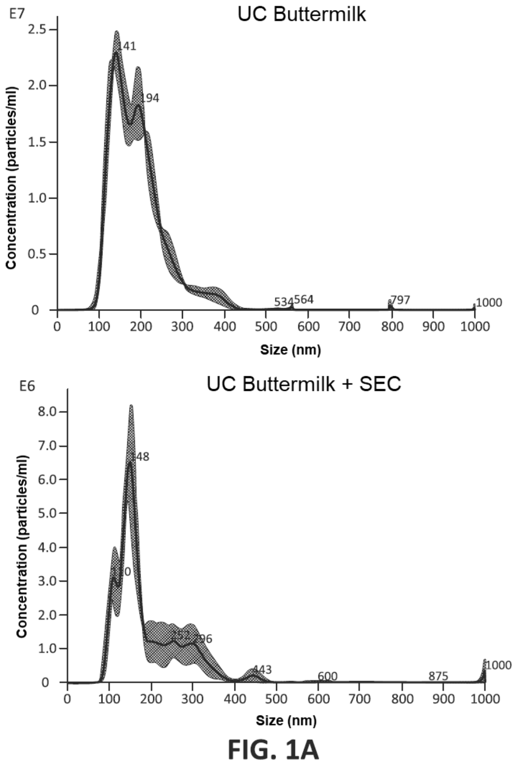

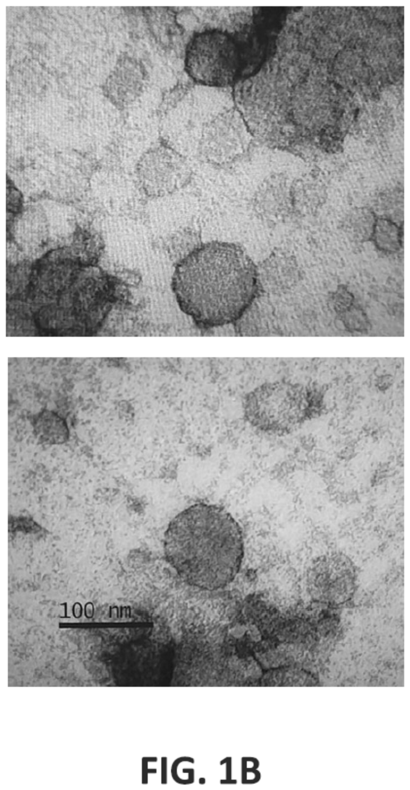

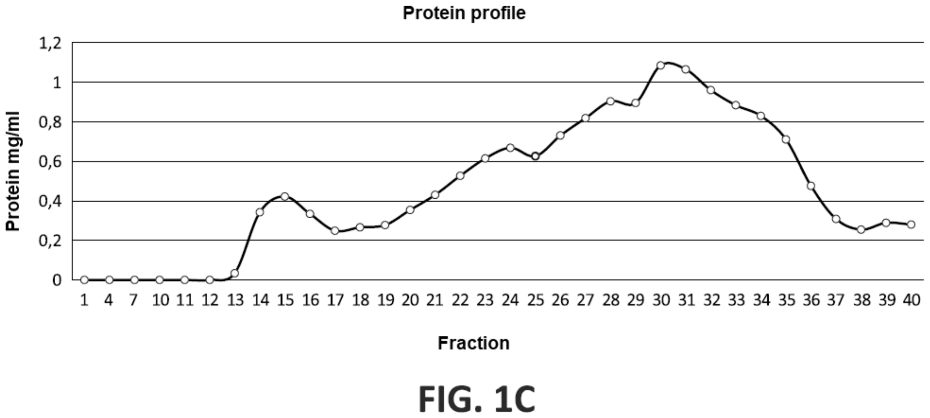

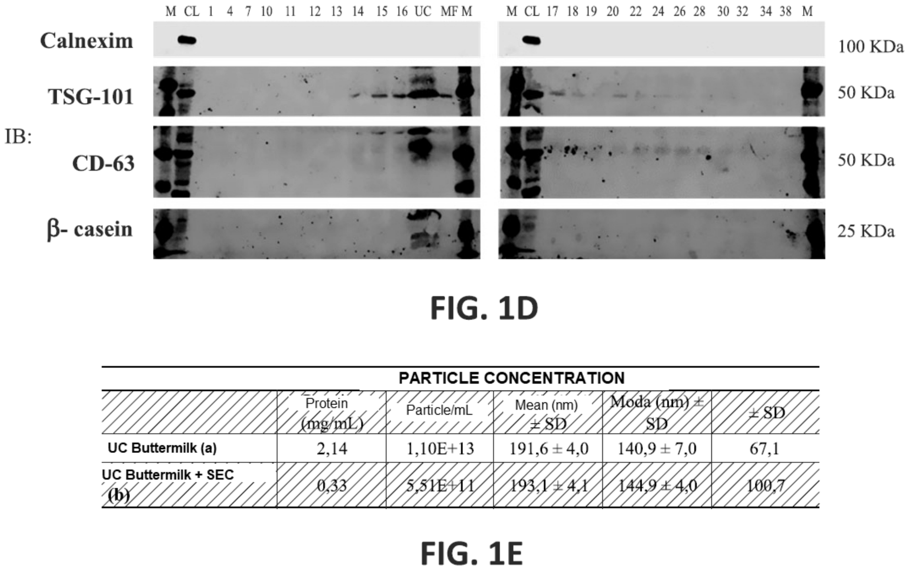

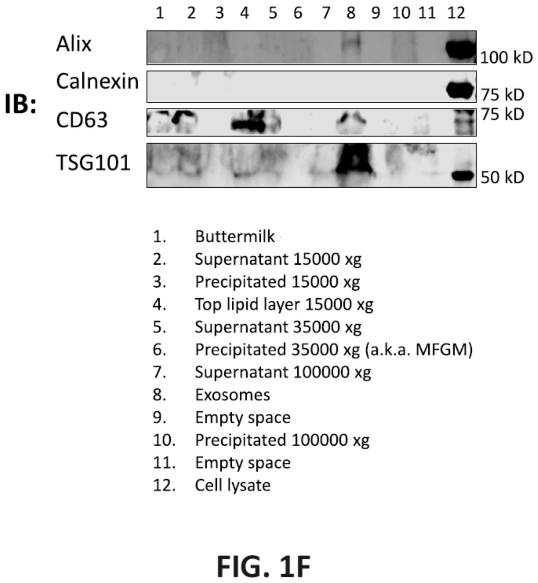

**Figure 1****: (A)** Size distribution profile of EVs from buttermilk isolated by one cycle of ultracentrifugation (UC) and ultracentrifugation and purification by size exclusion chromatography (UC+ SEC), obtained by NTA (Nanoparticle Tracking Analysis); **(B)** TEM images of extracellular vesicles isolated with UC+SEC; (C) Protein elution profile of extracellular vesicles isolated from buttermilk with UC combined with SEC; **(D)** Western blot analysis of proteins present (Hsp90, CD63 and TSG101) or absent (calnexin) in extracellular vesicles and abundant in bovine milk (β-casein); **(E)** Concentration of exosomes in buttermilk obtained by UC (a) or UC+SEC (b); **(F)** Protein analysis by Western Blot of exosome markers (1: Buttermilk); 2: Supernatant 15000 g; 3: Precipitate 15000 g; 4. Upper lipid layer 15000 g; 5. Supernatant 35000 g; 6. Precipitate 35000 g (aka MFGM); 7. Supernatant 100000 g; 8. Exosomes; 9. Empty space; 10. Precipitate 100000 g; 11. Empty space; 12. Cell lysate).

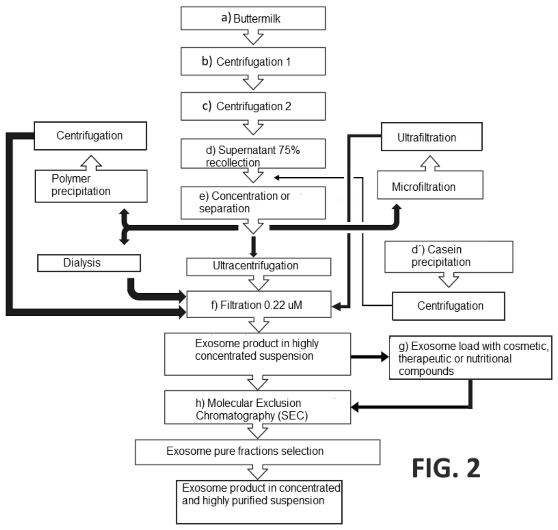

**Figure 2****:** Flow diagram of the exosome isolation procedure from buttermilk.

### Description of the invention

In response to the problems and needs of the state of the art, the authors of the present invention have directed their efforts to find a low-cost byproduct of the food industry, low in fat content, reduced in casein content and high in exosomes, allowing carrying out a new, simplified and more economical method for the extraction of exosomes and their scaling up to an industrial level.

Thus, after carrying out relevant experimental work, they have found that the material that meets these conditions is buttermilk, a byproduct derived from the dairy industry. Likewise, taking advantage of the low fat content of buttermilk and the reduced casein content, the authors of the invention have developed a simplified method for extracting exosomes specifically from this raw material.

Based on the above, the present invention refers to a new procedure for obtaining and isolating exosomes, of dairy origin, from a particular byproduct of the dairy industry such as buttermilk.

Exosomes, as contemplated in the present invention, are extracellular vesicles of endocytic origin, with a variable size between 40-150 nm, a density between 1.15-1.19 g/mL, a lipid bilayer structure in which a set of proteins are inserted, with various functions such as ligand or enzymatic functions, and that constrict a lumen in which a plethora of elements with biological function are transported.

In the context of the present invention, the term "buttermilk" refers to the whey obtained from the manufacture of butter, and can be defined as the aqueous phase released during the churning of the cream in the production of butter, and which contains all the soluble components of cream, such as proteins, lactose, minerals and phospholipids.

Buttermilk is a substrate especially rich in polar lipids (it is very low in fat, since the fat remains in the butter) and curiously the polar lipids are the phospholipids that are the raw material of exosomes. In fact, the buttermilk fraction contains exosomes with a different lipid profile than whole milk, whey or other byproduct of the dairy industry. This is because it starts from a fraction already enriched only in polar lipids. Since exosomes are dissolved in an aqueous phase, their composition will be a little different than other milk exosomes. Furthermore, when the fat globule breaks down, it releases phospholipids and, in general, there is usually a transfer of lipids from one system (exosome) to the other (aqueous solution of the buttermilk rich in phospholipids).

The procedure being object of the present invention is fundamentally based on physical separation techniques, such as centrifugation, and purification, using size exclusion chromatography, applied to the buttermilk as a specific starting material.

Thus, in a main aspect of the invention, a procedure (hereinafter, procedure of the invention) is contemplated for the isolation of exosomes from a byproduct of the dairy industry characterized in that it comprises the following stages:
(a) selecting buttermilk as a byproduct of the dairy industry,
(b) carrying out a first centrifugation (centrifugation 1) of the buttermilk obtained in (a), under centrifugal force conditions between 10,000 g and 15,000 g, at a temperature between 1°C and 8°C and for a time between 15 and 30 min, to obtain a fatty upper layer (fatty fraction), an aqueous fraction, and a precipitate;
(c) subjecting the aqueous fraction from stage (b) to a second centrifugation (centrifugation 2), under centrifugal force conditions between 30,000 g and 35,000 g, at a temperature between1 and 10°C and for a time between 50 min and 80 min; to obtain a precipitate and a supernatant;
(d) discarding the precipitate and collect the upper 75% of the supernatant obtained in c);
(e) concentrating or separating the exosomes from the supernatant fraction collected in d); and
(f) filtering the fraction obtained in e) to obtain a fraction enriched in exosomes.

The buttermilk selected as the starting product in the procedure of the invention can be freshly harvested buttermilk, as a byproduct of the industrial butter-making process, or buttermilk stored in freezing. The authors of the invention have demonstrated that buttermilk can be frozen for later use in a procedure to obtain exosomes without them undergoing significant changes.

Next, the mixture obtained is subjected to a differential centrifugation operation (stage b). This centrifugation stage can be carried out both discontinuously, in a fixed rotor centrifuge, and, preferably for industrial application, in a disc centrifuge. After this first centrifugation, 3 phases are obtained. An upper layer with the residual content of the buttermilk fat, an aqueous fraction (majority) that contains exosomes, and a precipitate in which foreign bodies, macroscopic fragments of tissues and cells reside. From this operation, the aqueous fraction is collected and the fatty fraction and the precipitate are discarded.

The aqueous fraction obtained from the previous stage (b) is subjected to a second centrifugation stage (c). After this operation, two phases are obtained: A minor precipitate, which is discarded, and an aqueous supernatant with a concentration gradient. At this point, preferably and advantageously for the procedure of the invention, 75% of the upper phase is collected (stage d). Although it is possible to use the entire supernatant, the authors of the present invention have verified that collecting only the upper 75% is a way to eliminate a good percentage of caseins that will influence all subsequent stages.

The supernatant fraction thus obtained is subjected to a separation or concentration operation of the exosomes (stage e).

In a particular embodiment of the procedure of the invention, stage e) consists of isolating the exosomes by means of a differential ultracentrifugation operation. This process is carried out by centrifuging the supernatant, obtained in the previous stage (d), under conditions of centrifugal force between 75,000 g and 125,000 g, for a time between 70 min and 2h, and at a temperature between 1°C and 10°C. After centrifugation, 4 phases are obtained. A gel-shaped pellet rich in caseins, a dense phase on the gel enriched in exosomes, an aqueous supernatant, and a thin upper layer rich in phospholipids. The dense phase rich in exosomes is selected, discarding all the content of the other fractions. This dense fraction is subjected to a filtration stage (f), preferably by using a filter with a pore size of 0.22 µm.

This fraction per se can already be used as a source of nanocarriers for different industrial applications such as, for example, in the field of the pharmaceutical, cosmetic and food industries.

In another particular embodiment, in stage e) the exosomes are separated by a combination of two tangential filtration operations, consisting of a first microfiltration and a second ultrafiltration. Microfiltration is carried out by filtering the supernatant, obtained in d), using a polypropylene membrane or cartridge, with a pore size between 800 nm and 1,200 nm, with a pressure between 0.5 bar and 3 bar and a flow between 100 L/m2h and 200 L/m2 h, up to 90% of the initial volume. Preferably, microfiltration is carried out with a polypropylene spiral membrane, with a pore size of 1 µm, at 1.5 bar and a flow of 150 L/m2h, until a concentration of 90% is reached.

The second stage of tangential filtration (ultrafiltration) is carried out in a hollow fiber membrane, made of any of the available hydrophilic materials, with a MWCO (Molecular Weight Cut-Off) between 200 KD and 700 kD, at a pressure between 1 .5 bar and 20 bar and with a flow between 20 L/m² h and 150 L/m² h. Preferably, the hollow fiber membrane is made of polyethersulfone, with a MWCO (Molecular Weight Cut-Off) of 500 kD, at a pressure of 17 bar and a flow of 50 L/m² h.

In another particular embodiment, stage e) consists of concentrating the exosomes from the supernatant obtained in d) by reverse dialysis. In a preferred embodiment, reverse dialysis is carried out with polyethylene glycol in a ratio of 8 to 12%, using a 10KDA membrane, at a temperature between 1 and 10°C and a time between 15 and 30 h.

In another particular embodiment, stage e) consists of separating the exosomes by precipitation with hydrophilic polymers, preferably food-grade, both in a monophasic system (only one polymer) or biphasic system (two polymers with different degrees of hydrophilicity). Preferably, the hydrophilic food-grade polymer used is polyethylene glycol. The supernatant obtained in d) is mixed with polyethylene glycol of molecular weight between 3,000 D and 10,000 D, in ratios between 5% (w/w) and 15% (w/w). Next, the mixture is kept gently stirring for a period between 1 and 10 min, after which it should be allowed to rest for between 1 h and 15 h. Next, gentle centrifugation is carried out under conditions between 6,000 g and 10,000 g, at a temperature between 1°C and 8°C and for a time between 10 min and 40 min, obtaining a precipitate highly enriched in exosomes.

In a preferred embodiment, precipitation is carried out by mixing the supernatant obtained in d) with polyethylene glycol 6000, in a ratio of 10% (w/w). Next, the mixture is kept gently stirring for 3 min and then allowed to rest for 8 h. Next, gentle centrifugation is performed at 8,000 g, at a temperature of 4°C and for 30 min.

In another particular embodiment, the precipitation can be carried out using a combination of two food-grade hydrophilic polymers, with a percentage of the mixture for the first of them between 95 and 80% (w/w) and for the second between 5% and 20% (w/w).

In a particular embodiment of the procedure of the invention, before stage e), and subsequently to stage d), a previous separation stage can be carried out by chemical precipitation and centrifugation (stage d'). For this, any acid, preferably food-grade (e.g. acetic or hydrochloric acid) is added to the supernatant fraction obtained in d) until a pH between 4.0 and 4.5 is reached. Once the desired pH has been reached, it should be left gently agitated or resting for a period of time between 10 min and 30 min and at a temperature between 20°C and 30°C. After this, the precipitation of the caseins occurs. These are separated from the aqueous phase containing the exosomes by an additional centrifugation operation under conditions between 2000 g and 8000 g, at a temperature between 1°C and 8°C and for a time between 10 min and 30 min. After this operation, a precipitate is obtained, which is discarded, containing mainly caseins and a supernatant in which exosomes are present.

**In** a preferred embodiment, chemical precipitation is carried out by adding hydrochloric acid until a pH of 4.5 is reached.

The fraction obtained in e), by any of the procedures described for concentrating or separating exosomes, is subjected to a filtration stage (stage f) to obtain a highly concentrated fraction of exosomes in suspension. Preferably, the filter used has a pore size of 0.22 µm.

The fraction enriched in exosomes obtained in f) can be preserved, until use, by freezing or, alternatively, after adding a cryopreservative. The exosome-enriched suspension can also be dried to obtain powdered exosomes for further use.

**In** order to obtain an even purer product, designed for applications that require the total elimination of other impurities that may be present, the fraction enriched in exosomes obtained in stage f), can be subjected to an additional purification stage by means of use of molecular exclusion chromatography (stage h). In particular embodiments, the process consists of using a column packed with Sepharose CL2B or other types of packing, such as Sephacril S-500, specifically for other types of applications that require isolating a different size of exosomes. Depending on the subsequent application to be used, the columns used can be of a wide range of sizes. For example, 30 cm x 300 mm, although it is also possible to use larger columns, 50 cm x 150 mm, with similar results. Depending on the column size, the exosome-enriched fractions may move slightly.

From the different fractions obtained after the purification of stage h), collected by elution with phosphate buffer, the pure fractions in exosomes are identified and selected, by measuring exosomal membrane markers, and they are mixed, giving rise to a concentrated and highly purified suspension exosome product, which is stored for later use.

The vesicle content of buttermilk is similar to an unprocessed product such as milk; however, the production procedure allows the elimination of different contaminants (e.g. fat) that interfere with the isolation. In comparative terms, buttermilk has a higher yield of vesicles than other byproducts of the food industry (e.g. whey).

In another main aspect of the invention, exosomes obtained from buttermilk are contemplated, or a product or composition that comprises said exosomes, isolated by the procedure of the invention.

These exosomes have the characteristics described in Figures **1A-1F****,** which can be summarized as: i) double lipid membrane (Fig. 1B); ii) size between 100 and 200 nm (Figures 1A, 1B, 1E); iii) presence of exosome-specific markers including TSG101, CD63 and Alix (Figure F); iv) absence of cell membrane markers (endoplasmic reticulum membrane), including Calnexin (Figure 1F); and v) absence of dairy casein (Figure 1D) (contaminant for the final exosome product).

The applicability of exosomes isolated from buttermilk are multiple, one of them being as nanocarriers of therapeutic, cosmetic or nutritional compounds, allowing their use in the field of Medicine, Cosmetics and Nutrition.

Thus, in a particular embodiment of the procedure of the invention, the fraction of isolated exosomes obtained in stage f), before the last purification by size exclusion chromatography (stage h), is subjected to an additional stage g), in that therapeutic, cosmetic or nutritional agents are incorporated into the exosomes. In particular embodiments, exosomes are loaded with RNA molecules. To do this, a volume of the exosome fraction obtained in f) is incubated with a solution of lipofectant agent (e.g. lipofectamine) and the RNA of interest for a time between 30 min and 60 min, at room temperature. (20-30°c). Excess RNA or other transfection reagents are subsequently removed during the purification process by size exclusion chromatography (stage h).

In other particular embodiments, exosomes are loaded by direct contact with a bioactive compound. In order to do this, a volume of the exosome fraction obtained in f) is incubated with a solution of a bioactive compound of phenolic nature dissolved in 5% ethanol/PBS for a time between 60 min and 120 min, at 37°C in the dark. The excess of unincorporated bioactive compound or other solvents is subsequently removed during the purification process by size exclusion chromatography (stage h).

### EXAMPLES

The present invention is further illustrated by the following examples, which are not intended to be limiting of its scope.

### EXAMPLE 1. Procedure to isolate exosomes from butter of bovine origin

Approximately 1 L of buttermilk (whey from the manufacturing of butter) of bovine origin was taken. This material was kept under refrigeration conditions (T = 4°C) until processing. The processing of the buttermilk was carried out within 24 h after obtaining it.

The buttermilk was centrifuged at 15,000 g for 30 min and at 4 °C, and the supernatant was collected. This was centrifuged again for 60 min at 35,000 g, at a temperature of 4°C. Next, ultracentrifugation was carried out at 100,000 g, for 1h 45 min, at 4°C, recovering the fraction rich in exosomes. This fraction was filtered with a filter with a pore size of 0.22 µm. Next, the filtrate obtained was subjected to purification by molecular exclusion chromatography (Figure 1C). The fraction purified in exosomes corresponds to fractions F14, F15, and F16 (Figure 1C). In the different fractions obtained, the fraction enriched in exosomes was identified by measuring exosomal membrane markers (Figure 1D). Said fractions are those fractions that are positive for the protein markers of TSG101, CD63 and free of the protein contaminants casein or calnexin. The fractions enriched in exosomes were mixed and stored at -80°C until further use. The electron microscopy images (Figure 1B), as well as the nanoparticle analysis (Figure 1A) confirmed that both the buttermilk and the fractions of the buttermilk purified with SEC, have vesicles of size between 100 and 200 nm (Figure 1B). It should be noted that during the purification process by size exclusion chromatography the number of particles was diluted, but the size was more homogeneous (Figure 1A and 1E). To differentiate exosomes from other lipid particles present in buttermilk (e.g., milk fat globule membranes, MFGM), their composition was analyzed for exosome-specific markers (Figure 1F). It was observed that the fraction precipitated after centrifugation at 35,000 g (fraction enriched in MFGM, lane 6), has no reactivity for the Alix, TSG101 or CD63 proteins; while the exosome fraction (Figure 1F, lane 8) are highly enriched in those proteins.

### EXAMPLE 2. Procedure to isolate exosomes from goat origin buttermilk

Approximately 1 L of buttermilk (whey from the manufacturing of butter) of goat origin was taken. This material was kept under refrigeration conditions (T=4°C) until processing. The processing of the buttermilk was carried out within 24 h after obtaining it.

The buttermilk was subjected to centrifugation at 15,000 g for 30 min and at 4 °C, after which the supernatant was collected. Hydrochloric acid was added to this supernatant until a pH of 4.50 was reached. The mixture thus obtained was left to gently stir for 25 min at room temperature. Next, centrifugation was carried out at 7,000 g, for 30 min, at 4°C, recovering the supernatant. This stage (acidification) is an optional stage, described in Figure 2 (stage d' [casein precipitation]) that can be performed at any stage before the concentration or separation process (stage e, Figure 2).

Next, ultracentrifugation was carried out at 100,000 g, for 1h 45 min, at 4°C, recovering the fraction rich in exosomes. This fraction was filtered with a filter with a pore size of 0.22 µm. This collected fluid was stored at -80°C until further use.

### EXAMPLE 3. Procedure to isolate exosomes from butter of bovine origin

Approximately 1 L of buttermilk (whey from the manufacturing of butter) of bovine origin was taken. This material was kept under refrigeration conditions (T=4°C) until processing. The processing of the buttermilk was carried out within 24 h after obtaining it.

The buttermilk was centrifuged at 15,000 g for 30 min and at 4 °C, and the supernatant was collected. This was centrifuged again for 60 min at 35,000 g and at a temperature of 4°C. Next, the exosomes were concentrated to 10% by reverse dialysis with 10% polyethylene glycol. Next, a subsequent purification process was carried out using size exclusion chromatography. The process consisted of using a 50 cm x 300 mm column filled with Sepharose CL2B and using saline phosphate buffer as the mobile phase. Next, 40 fractions of 3 mL each were collected, eluted with phosphate buffer (PBS). The exosome-enriched fractions were mixed and stored until further use.

### EXAMPLE 4. Procedure to isolate exosomes from butter of bovine origin

Approximately 1 L of buttermilk (whey from the manufacturing of butter) of bovine origin was taken. This material was kept under refrigeration conditions (T=4°C) until processing. The processing of the buttermilk was carried out within 24 h after obtaining it.

The buttermilk was subjected to centrifugation at 15,000 g for 30 min and at 4 °C, after which the supernatant was collected. Hydrochloric acid was added to this supernatant until a pH of 4.50 was reached. The mixture thus obtained was left to gently stir for 25 min at room temperature. Next, centrifugation was carried out at 7,000 g, for 30 min, at 4°C, recovering the supernatant. This stage (acidification) is an optional stage described in Figure 2 (stage d' [casein precipitation]) that can be performed at any stage before the concentration or separation process (stage e, Figure 2). Next, the exosomes present in the supernatant were concentrated by reverse dialysis with 10% polyethylene glycol. Next, a subsequent purification process was carried out using size exclusion chromatography. The process consisted of using a 50 cm x 300 mm column filled with Sepharose CL2B and using saline phosphate buffer as the mobile phase. Next, 40 fractions of 3 mL each were collected. The exosome-enriched fractions were mixed and stored until further use.

### EXAMPLE 5. Procedure to isolate exosomes from buttermilk of bovine origin for use as nanocarriers

Approximately 1 L of buttermilk (whey from the manufacturing of butter) of bovine origin was taken. This material was kept under refrigeration conditions (T=4°C) until processing. The processing of the buttermilk was carried out within 24 h after obtaining it.

The buttermilk was centrifuged at 15,000 g for 30 min and at 4 °C, and the supernatant was collected. This was centrifuged again for 60 min at 35,000 g, at a temperature of 4°C. Next, ultracentrifugation was carried out at 100,000 g, for 1h 45 min, at 4°C, recovering the fraction rich in exosomes. This fraction was filtered with a filter with a pore size of 0.22 µm.

The suspension highly enriched in exosomes thus obtained was contacted with a transfectant solution containing: i) a lipofectant agent (e.g., Lipofectamine RNAi max); ii) nucleic acids (e.g., human or plant miRNAs); and iii) the nucleic acid eluent (e.g., RNAi buffer or RNase- and DNase-free water) in a ratio of approximately 5:1 of the exosomes and transfectant solution. The mixture was incubated for 30 min at room temperature. The solution obtained was then subjected to additional purification by size exclusion chromatography. The process consisted of using a 50 cm x 300 mm column filled with Sepharose CL2B and using saline phosphate buffer as the mobile phase. In the different fractions obtained, the fraction enriched in exosomes was identified by measuring exosomal membrane markers. The fractions enriched in exosomes were mixed and stored at -80°C until further use.

RNA was extracted from the exosome-enriched samples and the incorporated miRNAs were quantified. An increase in the expression of miRNAs of more than 10,000 times was observed when a single miRNA was used (at a dose of 100 uM) or around 500 times when 5 miRNAs were used at the same time (at a dose of 20 uM each)..

The loaded exosomes were exposed to cell lines (e.g. intestinal Caco-2 or hepatic HepG2) and incubated at a concentration of 100 µg of exosome protein/mL of culture medium for 24 h and their uptake was verified. After 24 h of exposure to loaded exosomes (with 5 miRNAs at a time), the cells increased the levels of the tested miRNAs up to 20 times their initial value.

## Claims

1. - Procedure for isolating exosomes from a byproduct of the dairy industry **characterized in that** it comprises the following stages:
(a) selecting buttermilk as a byproduct of the dairy industry,
(b) carrying out a first centrifugation of the buttermilk obtained in (a), under conditions of centrifugal force between 10,000 g and 15,000 g, at a temperature between 1°C and 8°C and for a time between 15 and 30 min, to obtain a fatty upper layer, an aqueous fraction, and a precipitate;
(c) subjecting the aqueous fraction from stage (b) to a second centrifugation, under centrifugal force conditions between 30,000 g and 35,000 g, at a temperature between 1 and 10°C and for a time between 50 min and 1 hour and 20 min; to obtain a precipitate and a supernatant;
(d) collecting the upper 75% of the supernatant obtained in c);
(e) concentrating or separating the exosomes from the supernatant fraction collected in d); and
(f) filtering the fraction obtained in e) to obtain a fraction enriched in exosomes.

2. Procedure for isolating exosomes according to claim 1 **characterized in that** the buttermilk selected in a) can be freshly obtained buttermilk as a byproduct of the industrial process of butter production or b) frozen buttermilk.

3. Procedure for isolating exosomes according to claim 1 **characterized in that** stage e) is carried out by means of differential ultracentrifugation, under conditions of centrifugal force between 75,000 and 125,000 g, for a time between 1 and 2 h, and at a temperature between 1 and 10°C, to obtain a gel-shaped pellet; a dense exosome-rich phase on the gel, an aqueous supernatant, and a thin upper layer rich in phospholipids.

4. Procedure for isolating exosomes according to claim 1 **characterized in that** stage e) is carried out by means of a combination of two tangential filtration operations comprising microfiltration followed by ultrafiltration.

5. Procedure for isolating exosomes according to claim 4 **characterized in that** the microfiltration comprises the filtration of the supernatant obtained in d) through the use of a polypropylene membrane, with a pore size between 800 and 1,200 nm, with a pressure between 0.5 and 3 bar and a flow between 100 and 200 L/m² h, until reaching a concentration of 90%.

6. Procedure for isolating exosomes according to claim 5 wherein the filtration is carried out with a polypropylene spiral membrane, with a pore size of 1 µm, at 1.5 bar and a flow of 150 L/m² h until a concentration of 90% is reached.

7. Procedure for isolating exosomes according to claim 5 or 6, **characterized in that** the ultrafiltration is carried out in a hollow fiber membrane, with a MWCO (Molecular Weight Cut-Off) between 200 and 700 kD, at a pressure between 1.5 and 20 bar and with a flow between 20 and 150 L/m² h.

8. Procedure for isolating exosomes according to claim 7 wherein the hollow fiber membrane is a hollow fiber polyethersulfone membrane, with a MWCO (Molecular Weight Cut-Off) of 500 kD, at a pressure of 17 bar and a flow of 50 L/m² h.

9. Procedure for isolating exosomes according to claim 1 wherein stage e) comprises a precipitation with one or more hydrophilic polymers, followed by centrifugation.

10. Procedure for isolating exosomes according to claim 9 wherein the precipitation is carried out using polyethylene glycol.

11. Procedure for isolating exosomes according to claim 10 wherein:
- the supernatant obtained in d) is mixed with polyethylene glycol with a molecular weight between 3,000 and 10,000 D, in ratios between 5 and 15% (w/w),
- the mixture obtained in the previous stage is kept under gentle stirring for a period between 1 and 10 min and is subsequently allowed to rest for between 1 and 15 h, and
- after resting, the mixture is subjected to gentle centrifugation under conditions between 6,000 and 8,000 g, at a temperature between 1 and 8°C, and for a time between 10 and 40 min, to obtain a highly concentrated precipitate enriched in exosomes.

12. Procedure for isolating exosomes according to claim 11 wherein:
- the supernatant obtained in d) is mixed with polyethylene glycol 6000, in a ratio of 10% (w/w),
- the mixture obtained in the previous stage is kept gently stirring for 3 min and then allowed to rest for 8 h, and
- after resting, the mixture is subjected to gentle centrifugation at 8,000 g, at a temperature of 4°C, and for 30 min.

13. Procedure for isolating exosomes according to claim 9 wherein the precipitation is carried out using a combination of two food-grade hydrophilic polymers with a percentage of the mixture for the first of them between 95 and 80% (w/w) and for the second of them between 5% and 20% (p/p).

14. Procedure for isolating exosomes according to claim 1 wherein stage e) is carried out by reverse dialysis.

15. Procedure for isolating exosomes according to claim 14 wherein the reverse dialysis is carried out with polyethylene glycol in a ratio of 8 to 12%.

16. Procedure for isolating exosomes according to any of the preceding claims, **characterized in that** a stage d'), prior to stage e), is optionally carried out, which comprises a chemical precipitation followed by centrifugation to obtain a precipitate that mainly comprises caseins and a supernatant comprising the exosomes.

17. Procedure for isolating exosomes according to claim 16 **characterized in that** stage d') comprises:
- subjecting the supernatant fraction obtained in d) to a chemical precipitation by adding an acid until reaching a pH of between 4.0 and 4.5 and keeping it under gentle stirring or resting for a period of time between 10 and 30 min and at a temperature between 20°C and 30°C, and
- subjecting the liquid fraction from the previous stage to a centrifugation operation under conditions between 2,000 g and 8,000 g, at a temperature between 1°C and 8°C, and for a time between 10 and 30 min.

18. Procedure for isolating exosomes according to claim 17 wherein the acid used is a food-grade acid.

19. Procedure for isolating exosomes according to claim 18 wherein the chemical precipitation is carried out by adding hydrochloric acid until a pH of 4.5 is reached.

20. Procedure for isolating exosomes according to any of the preceding claims wherein the filtration of stage f) is carried out using a filter with a pore size of 0.22 µm.

21. Procedure for isolating exosomes according to any of the preceding claims wherein, optionally, in an additional stage g) therapeutic, cosmetic or nutritional agents are incorporated into the exosomes obtained in f).

22. Procedure for isolating exosomes according to claim 21 wherein stage g) is carried out by incubating the exosomes obtained in f) with a solution of lipofectant agent and a nucleic acid molecule, for a time between 30 and 60 min, at room temperature, or by direct contact.

23. Procedure for isolating exosomes according to any of the preceding claims wherein the fraction enriched in exosomes obtained in stage f) or g) is subjected to an additional purification stage h) by using molecular exclusion chromatography.

24. Exosomes isolated from buttermilk by the procedure of any of claims 1-23.

25. Use of the isolated exosomes according to claim 24 as nanocarriers of compounds.

26. Use of isolated exosomes, according to claim 25, wherein the exosomes comprise therapeutic, cosmetic or nutritional compounds incorporated therein.

27. Exosomes according to claim 24 for their use in medicine.
